(19) Europäisches Patentamt
European Patent Office
Office européen des brevets

(11) Veröffentlichungsnummer: **0 267 435 A1**

# (12) EUROPÄISCHE PATENTANMELDUNG

(21) Anmeldenummer: **87114816.9**

(22) Anmeldetag: **10.10.87**

(51) Int. Cl.⁴: **C07D 409/14**, C07D 409/06, C07D 413/14, A01N 43/28, A01N 43/40, A01N 43/84

(30) Priorität: **21.10.86 DE 3635744**

(43) Veröffentlichungstag der Anmeldung: **18.05.88 Patentblatt 88/20**

(84) Benannte Vertragsstaaten: **AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Weissmüller, Joachim, Dr.**
**Carl-Langhans-Strasse 53**
**D-4019 Monheim(DE)**
Erfinder: **Krämer, Wolfgang, Dr.**
**Rosenkranz 25**
**D-5093 Burscheid 2(DE)**
Erfinder: **Berg, Dieter, Dr.**
**Gellertweg 27**
**D-5600 Wuppertal 1(DE)**
Erfinder: **Reinecke, Paul, Dr.**
**Steinstrasse 8**
**D-5090 Leverkusen 3(DE)**
Erfinder: **Brandes, Wilhelm, Dr.**
**Eichendorffstrasse 3**
**D-5653 Leichlingen(DE)**

## (54) Substituierte Aminoketale.

(57) Substituierte Aminoketale der Formel (I)

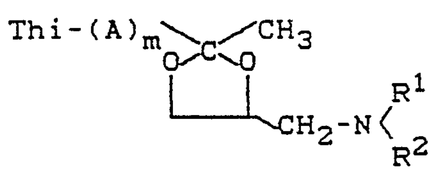

(I)

in welcher
Thi, A, m, $R^1$ und $R^2$ die im Text angegebenen Bedeutungen haben und ihre Verwendung als Schädlingsbekämpfungsmittel.

Die neuen substituierten Aminoketale der Formel (I) können z.B. erhalten werden, wenn man geeignete Dioxolane mit geeigneten Aminen umsetzt oder geeignete durch einen Thienylalkenylrest substituierte Dioxolane hydriert.

Weiterhin Dioxolane der Formel (II),

EP 0 267 435 A1

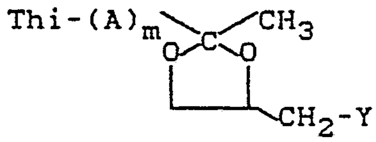

.(II)

in welcher

Thi, A, m und Y die in der Beschreibung gegebene Bedeutung haben und geeignete Verfahren zu ihrer Herstellung, z.B. aus geeigneten Ketonen durch Umsetzung mit geeigneten Diolen.

## Substituierte Aminoketale

Die Erfindung betrifft neue substituierte Aminoketale, mehrere Verfahren zu ihrer Herstellung und ihre Verwendung als Schädlingsbekämpfungsmittel.

Es ist bereits bekannt, daß bestimmte substituierte Aminoketale, wie beispielsweise das 2-[1-(4-Chlorphenyl)-2-methylprop-2-yl]-4-(3,5-dimethylpiperidin-1-yl-methyl)-2-methyl-dioxolan oder das 4-(3,5-Dimethylpiperidin-1-yl-methyl)-2-[1-(4-fluorphenyl)-2-methylprop-2-yl]-4-methyl-1,3-dioxolan fungizide Eigenschaften besitzen (vgl. EP 97 822).

Deren Wirksamkeit ist jedoch, insbesondere bei niedrigen Aufwandmengen und Konzentrationen, nicht in allen Anwendungsbereichen völlig zufriedenstellend.

Es wurden neue substituierte Aminoketale der allgemeinen Formel (I),

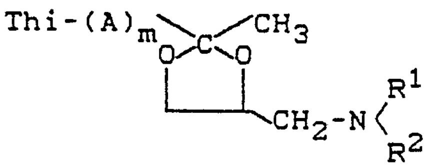

(I)

in welcher
Thi für gegebenenfalls substituiertes Thienyl steht,
A für eine Alkylen-oder Alkenylenkette steht,
m für eine Zahl 0 oder 1 steht,
$R^1$ für Wasserstoff, Alkyl oder Alkenyl steht und
$R^2$ für Alkyl oder Alkenyl steht oder
$R^1$ und $R^2$ gemeinsam mit dem Stickstoffatom, an welches sie gebunden sind, für einen Rest

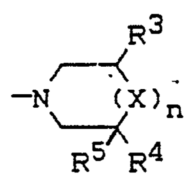

stehen, wobei
$R^3$, $R^4$ und $R^5$ unabhängig voneinander jeweils für Wasserstoff, Alkyl oder Hydroxyalkyl stehen,
X für Sauerstoff, Schwefel, eine Methylen-oder eine Ethylengruppe steht und
n für eine Zahl 0 oder 1 steht,
sowie deren pflanzenverträgliche Säureadditionssalze und Metallsalzkomplexe gefunden.

Die Verbindungen der Formel (I) können als geometrische und/oder optische Isomere oder Isomerengemische unterschiedlicher Zusammetsetzung anfallen. Sowohl die reinen Isomeren als auch die Isomerengemische werden erfindungsgemäß beansprucht.

Weiterhin wurde gefunden, daß man die neuen substituierten Aminoketale der allgemeinen Formel (I),

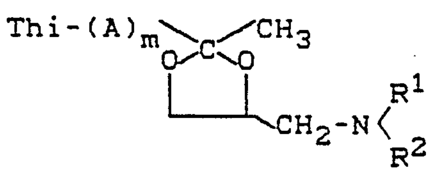

(I)

in welcher
Thi für gegebenenfalls substituiertes Thienyl steht,
A für eine Alkylen-oder Alkenylenkette steht;
m für eine Zahl 0 oder 1 steht,
$R^1$ für Wasserstoff, Alkyl oder Alkenyl steht und
$R^2$ für Alkyl oder Alkenyl steht oder

$R^1$ und $R^2$ gemeinsam mit dem Stickstoffatom, an welches sie gebunden sind, für einen Rest

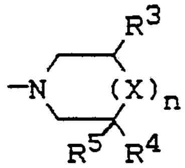 stehen, wobei

$R^3$, $R^4$ und $R^5$ unabhängig voneinander jeweils für Wasserstoff, Alkyl oder Hydroxyalkyl stehen,
X für Sauerstoff, Schwefel, eine Methylen-oder eine Ethylengruppe steht und
n für eine Zahl 0 oder 1 steht,
sowie deren pflanzenverträgliche Säureadditionssalze und Metallsalzkomplexe erhält, wenn man
(a) Dioxolane der Formel (II)

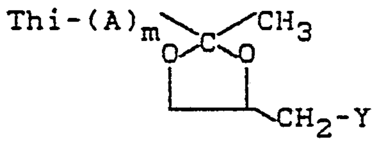 (II)

in welcher
Y für eine elektronenanziehende Abgangsgruppe steht und
Thi, A und m die oben angegebene Bedeutung haben,
mit Aminen der Formel (III),

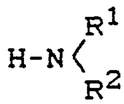 (III)

in welcher
$R^1$ und $R^2$ oben angegebene Bedeutung haben,
gegebenenfalls in Gegenwart eines Verdünnungsmittels und gegebenenfalls in Gegenwart eines Reaktionshilfsmittels umsetzt oder wenn man
(b) die mit Hilfe des Verfahrens (a) erhältlichen substituierten Aminoketale der Formel (Ia),

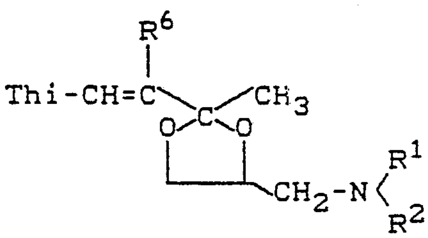 (Ia)

in welcher
$R^6$ für Wasserstoff oder Alkyl steht und
Thi, $R^1$ und $R^2$ die oben angegebene Bedeutung haben,
mit Wasserstoff in Gegenwart eines Katalysators und in Gegenwart eines Verdünnungsmittels hydriert und gegebenenfalls anschließend eine Säure oder ein Metallsalz addiert.

Schließlich wurde gefunden, daß die neuen substituierten Aminoketale der allgemeinen Formel (I) eine Wirkung gegen Schädlinge besitzen.

Überraschenderweise zeigen die erfindungsgemäßen substituierten Aminoketale der allgemeinen Formel (I) eine erheblich bessere fungizide Wirksamkeit als die aus dem Stand der Technik bekannten substituierten Aminoketale, wie beispielsweise das 2-[1-(4-Chlorphenyl)-2-methylprop-2-yl]-4-(3,5-dimethylpiperidin-1-yl-methyl)-2-methyl-dioxolan oder das 4-(3,5-Dimethylpiperidin-1-yl-methyl)-2-[1-(4-fluorphenyl)-2-methylprop-2-yl]-4-methyl-1,3-dioxolan, welches chemisch und wirkungsmäßig naheliegende Verbindungen sind.

Die erfindungsgemäßen substituierten Aminoketale sind durch die Formel (I) allgemein definiert. Bevorzugt sind Verbindungen der Formel (I), bei welchen

Thi für gegebenenfalls ein-oder zweifach, gleich oder verschieden substituiertes Thienyl steht, wobei als Substituenten infrage kommen: Halogen, Nitro, jeweils geradkettiges oder verzweigtes Alkyl, Halogenalkyl oder Halogenalkoxy mit jeweils 1 bis 4 Kohlenstoffatomen und gegebenenfalls 1 bis 9 gleichen oder verschiedenen Halogenatomen,

A für eine jeweils geradkettige oder verzweigte Alkylenkette oder Alkenylenkette mit jeweils 3 bis 6 Kohlenstoffatomen steht,

m für eine Zahl 0 oder 1 steht,

$R^1$ für Wasserstoff oder für jeweils geradkettiges oder verzweigtes Alkyl oder Alkenyl mit jeweils bis zu 12 Kohlenstoffatomen steht und

$R^2$ für jeweils geradkettiges oder verzweigtes Alkyl oder Alkenyl mit jeweils bis zu 12 Kohlenstoffatomen steht oder

$R^1$ und $R^2$ gemeinsam mit dem Stickstoffatom, an welches sie gebunden sind, für einen Rest

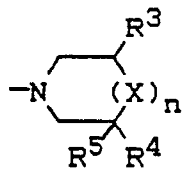 stehen, wobei

$R^3$, $R^4$ und $R^5$ unabhängig voneinander jeweils für Wasserstoff oder für jeweils geradkettiges oder verzweigtes Alkyl oder Hydroxyalkyl mit jeweils 1 bis 4 Kohlenstoffatomen stehen,

X für Sauerstoff, Schwefel, eine Methylen-oder eine Ethylengruppe steht und

n für eine Zahl 0 oder 1 steht.

Besonders bevorzugt sind Verbindungen der Formel (I), bei welchen

Thi für gegebenenfalls ein-oder zweifach, gleich oder verschieden substituiertes 2-oder 3-Thienyl steht, wobei als Substituenten in Frage kommen: Fluor, Chlor, Brom, Nitro, Methyl, Ethyl, n-oder i-Propyl, n-, i-, s-oder t-Butyl,

A für eine der Gruppen 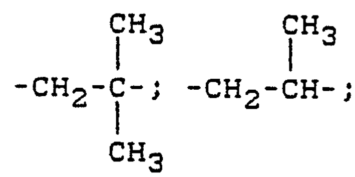

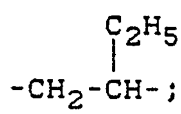 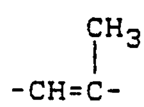 oder 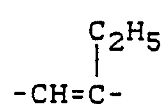 steht,

m für eine Zahl 0 oder 1 steht,

$R^1$ für Wasserstoff oder für jeweils geradkettiges oder verzweigtes Alkyl oder Alkenyl mit jeweils bis zu 6 Kohlenstoffatomen steht und

$R^2$ für jeweils geradkettiges oder verzweigtes Alkyl oder Alkenyl mit jeweils bis zu 6 Kohlenstoffatomen steht.

Weiterhin sind besonders bevorzugt Verbindungen der Formel (I), bei welchen

Thi für gegebenenfalls ein-oder zweifach, gleich oder verschieden substituiertes 2-odr 3-Thienyl steht, wobei als Substituenten infrage kommen: Fluor, Chlor, Brom, Nitro, Methyl, Ethyl, n-oder i-Propyl, n-, i-, s-oder t-Butyl,

A für eine der Gruppen 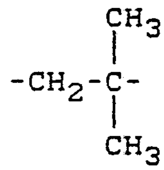 ; 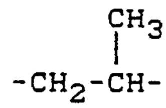 ;

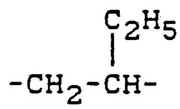 ; 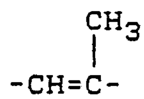 oder 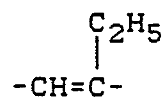 steht,

m für eine Zahl 0 oder 1 steht, und
$R^1$ und $R^2$ gemeinsam mit dem Stickstoffatom, an welches sie gebunden sind, für einen Rest

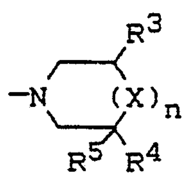

stehen, wobei
$R^3$, $R^4$ und $R^5$ unabhängig voneinander jeweils für Wasserstoff, Methyl, Ethyl oder Hydroxymethyl stehen,
X für Sauerstoff, Schwefel, eine Methylengruppe oder eine Ethylengruppe steht und
n für Zahl 0 oder 1 steht.

Im einzelnen seien außer den bei den Herstellungsbeispielen genannten Verbindungen, die folgenden substituierten Aminoketale der allgemeinen Formel (I) genannt:

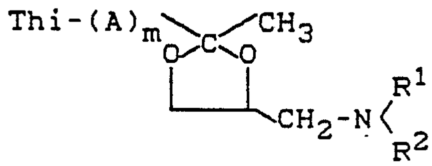

(I)

| Thi | A | m | $-N\langle^{R^1}_{R^2}$ |
|---|---|---|---|
| 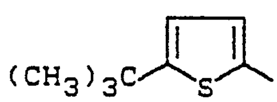 | - | 0 | 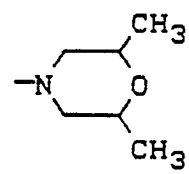 |
| 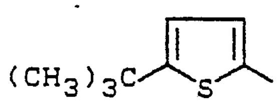 | - | 0 | 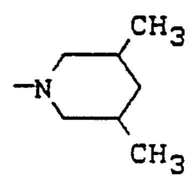 |
| 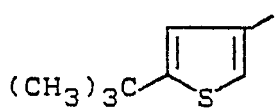 | - | 0 | 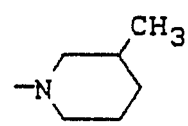 |
| 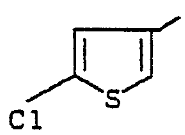 | 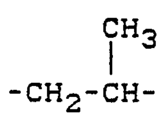 | 1 | 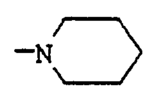 |

| Thi | A | m | 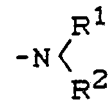 |
|---|---|---|---|
| 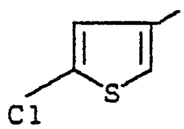 | 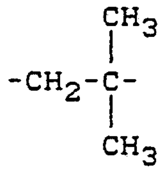 | 1 | 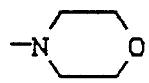 |
| 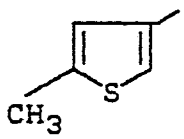 | 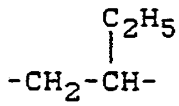 | 1 | 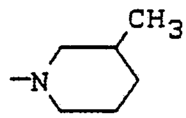 |
| 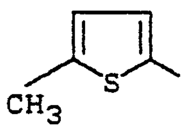 | 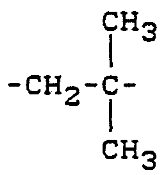 | 1 | 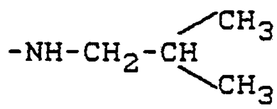 |
| 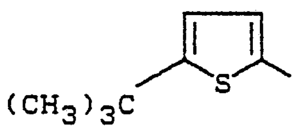 | 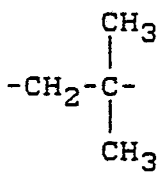 | 1 | 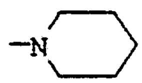 |
| 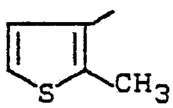 | 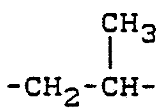 | 1 | 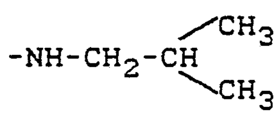 |

Verwendet man beispielsweise 4-Chlormethyl-2-[1-(3-methylthien-2-yl)-propen-2-yl]-2-methyl-1,3-dioxolan und 3-Methylpiperidin als Ausgangsstoffe, so läßt sich der Reaktionsablauf des erfindungsgemäßen Verfahrens (a) durch das folgende Formelschema darstellen:

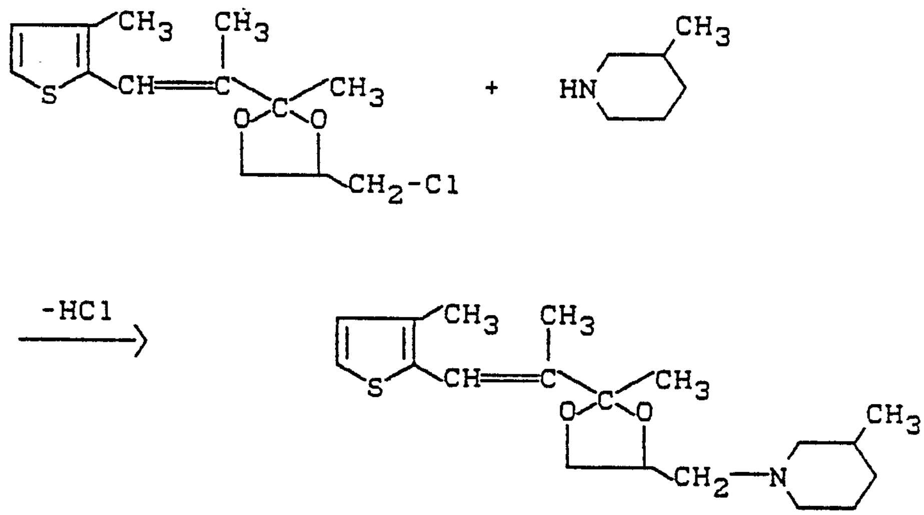


Verwendet man beispielsweise 4-(3-Methylpiperidin-1-yl-methyl)-2-[1-(3-methylthien-2-yl)-propen-2-yl]-2-methyl-1,3-dioxolan als Ausgangsverbindung, so läßt sich der Reaktionsablauf des erfindungsgemäßen Verfahrens (b) durch das folgende Formelschema darstellen:

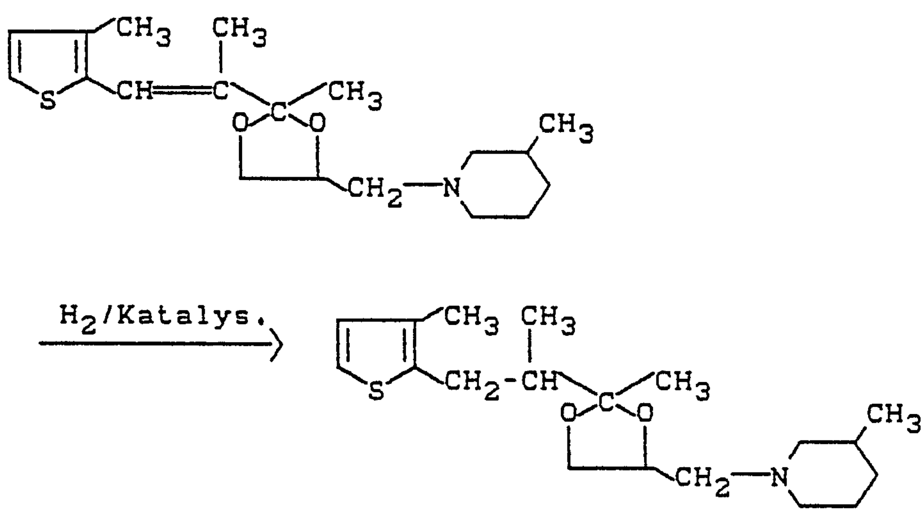


Die zur Durchführung des erfindungsgemäßen Verfahrens (a) als Ausgangsstoffe benötigten Dioxolane sind durch die Formel (II) allgemein definiert. In dieser Formel (II) stehen Thi, A und m vorzugsweise für diejenigen Zahlen und Reste, die bereits im Zusammenhang mit der Beschreibung der erfindungsgemäßen Stoffe der Formel (I) als bevorzugt für diese Substituenten und Indices genannt wurden.

Y steht vorzugsweise für Halogen, insbesondere für Chlor oder Brom, oder für gegebenenfalls substituiertes Alkylsulfonyloxy oder Arylsulfonyloxy, wie beispielsweise Methylsulfonyloxy oder p-Toluolsulfonyloxy.

Die Dioxolane der Formel (II)

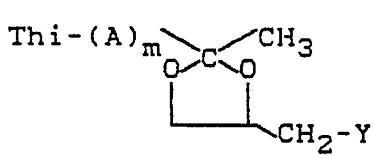
(II)

in welcher
Thi für gegebenenfalls substituiertes Thienyl steht,
A für eine Alkylen-oder Alkenylenkette steht,
m für eine Zahl 0 oder 1 steht und

Y für eine elektronenanziehende Abgangsgruppe steht, sind noch nicht bekannt und ebenfalls Gegenstand der Erfindung.

Man erhält sie jedoch in Analogie zu bekannten Verfahren (vgl. z.B. EP 97 822), beispielsweise wenn man Ketone der Formel (IV),

Thi-$(A)_m$- C -$CH_3$ (IV)
||
O

in welcher
Thi, A und m die oben angegebene Bedeutung haben, mit Diolen der Formel (V),

OH
|
HO-$CH_2$- CH -$CH_2$-$Y^1$ (V)

in welcher
$Y^1$ für Hydroxy oder Halogen steht,
gegenbenenfalls in Gegenwart eines Verdünnungsmittels, wie beispielsweise Toluol, und gegebenenfalls in Gegenwart eines Katalysators, wie beispielsweise p-Toluolsulfonsäure oder ein saurer Ionenaustauscher, bei Tempera turen zwischen 50 °C und 120 °C umsetzt, und wenn man in den Fällen, wo $Y^1$ in Formel (V) für eine Hydroxygruppe steht, in einer 2. Stufe die so erhältlichen Hydroxymethyldioxolane der Formel (VI),

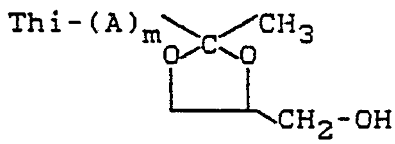
(VI)

in welcher
Thi, A und m die oben angegebene Bedeutung haben,
mit gegebenenfalls substituierten Alkyl-oder Arylsulfonylhalogeniden der Formel (VII),

Z-$SO_2$-Hal (VII)

in welcher
Hal für Halogen, insbesondere für Chlor steht und
Z für jeweils gegebenenfalls substituiertes Alkyl oder Aryl, wie insbesondere Methyl, Trifluormethyl oder 4-Methylphenyl steht,
gegebenenfalls in Gegenwart eines Verdünnungsmittels, wie beispielsweise Diethylether, und gegebenenfalls in Gegenwart eines Säurebindemittels, wie beispielsweise Pyridin oder Triethylamin, bei Temperaturen zwischen -20 °C und +100 °C umsetzt.

Die Ketone der Formel (IV) sind teilweise bekannt (vgl. z.B. Bull. Chem. Soc. Japan 49, 1369 - 1374 [1976], Bull. Soc. Chim. Fr. 1970, 1497-1502) oder erhältlich nach bekannten Verfahren [vgl. auch Ber. dtsch. chem. Ges. 17, 2643 (1884) sowie Ber. dtsch. chem. Ges. 19, 1859 (1886)], beispielsweise wenn man die allgemein bekannten Thiophenaldehyde der Formel (VIII),

Thi-CHO (VIII)

in welcher
Thi die oben angegebene Bedeutung hat,
mit Ketonen der Formel (IX),

$R^6$-$CH_2$- C -$CH_3$ (IX)
||
O

in welcher
$R^6$ für Wasserstoff oder Alkyl steht,
gegebenenfalls in Gegenwart eines Verdünnungsmittels, wie beispielsweise Diethylether, und gegebenenfalls in Gegenwart eines Katalysators, wie beispielsweise Chlorwasserstoffsäure, bei Temperaturen zwischen -20° C und +80 °C kondensiert (Aldolkondensation) oder wenn man ebenfalls in Analogie zu bekannten Verfahren (vgl. z.B. DE-OS 32 10 725) Halogenmethylthiophene der Formel (X),

Thi-$CH_2$-$Hal^1$ (X)

in welcher
Thi die oben angegebene Bedeutung hat und
$Hal^1$ für Halogen, insbesondere für Chlor oder Brom steht,
mit Ketonen der Formel (IXa),

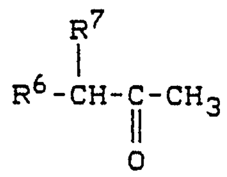 (IXa)

in welcher
$R^6$ für Wasserstoff oder Alkyl steht und
$R^7$ für Alkyl steht,
gegebenenfalls in Gegenwart eines Verdünnungsmittels, wie beispielsweise Hexan oder Toluol, und gegebenenfalls in Gegenwart einer Base, wie beispielsweise Kaliumhydroxid, sowie gegebenenfalls in Gegenwart eines Phasentransferkatalysators, wie beispielsweise Tetrabutylammoniumbromid bei Temperaturen zwischen +20 °C und +120 °C umsetzt.

Die Diole der Formel (V), die Alkyl-bzw. Arylsulfonylhalogenide der Formel (VII), die Thiophenaldehyde der Formel (VIII), die Ketone der Formel (IX) und (IXa) sowie die Halogenmethylthiophene der Formel (X) sind allgemein bekannte Verbindungen der organischen Chemie oder erhältlich in Analogie zu allgemein bekannten Verfahren.

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind ebenfalls allgemein bekannte Verbindungen der organischen Chemie.

Die zur Durchführung des erfindungsgemäßen Verfahrens (b) als Ausgangsstoffe benötigten substituierten Aminoketale sind durch die Formel (Ia) allgemein definiert. In dieser Formel (Ia) stehen Thi, $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden und
$R^6$ steht vorzugsweise für Wasserstoff oder für geradkettiges oder verzweigtes Alkyl mit 1 bis 4 Kohlenstoffatomen, insbesondere für Methyl und Ethyl.

Die substituierten Aminoketale der Formel (Ia) sind erfindungsgemäße Verbindungen und erhältlich mit Hilfe des erfindungsgemäßen Verfahrens (a).

Als Verdünnungsmittel zur Durchführung des erfindungsgemäßen Verfahrens (a) kommen inerte organische Lösungsmittel infrage.
Hierzu gehören insbesondere aliphatische oder aromatische Kohlenwasserstoffe, wie beispielsweise Benzin, Benzol, Toluol, Xylol, Petrolether, Hexan, Cyclohexan; Ether, wie Diethylether, Dioxan, Tetrahydrofuran oder Ethylenglykoldimethyl-oder diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide, wie Dimethylsulfoxid oder Alkohole, wie Propanol oder Butanol. Das erfindungsgemäße Verfahren (a) kann jedoch auch ohne Zusatz eines Verdünnungsmittels durchgeführt werden.

Das erfindungsgemäß Verfahren (a) kann gegebenenfalls in Gegenwart einer Base als Reaktionshilfsmittel durchgeführt werden.

Als solche kommen alle üblichen anorganischen oder organischen Basen in Frage. Hierzu gehören beispielsweise Alkalimetallhydroxide, wie Natriumhydroxid oder Kaliumhydroxid; Alkalimetallcarbonate, wie Natriumcarbonat, Kaliumcarbonat, oder Natriumhydrogencarbonat; sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, das als Reaktionspartner verwendete Amin der Formel (III) in entsprechendem Überschuß gleichzeitig als Reaktionshilfsmittel und/oder als Verdünnungsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 50 °C und 250 °C, vorzugsweise bei Temperaturen zwischen 80 °C und 200 °C.

Das erfindungsgemäße Verfahren (a) wird üblicherweise bei Normaldruck durchgeführt. Es ist jedoch auch möglich bei erhöhtem Druck zwischen 1,5 und 5,0 atü zu arbeiten.

Zur Durchführung des erfindungsgemäßen Verfahrens (a) setzt man pro Mol an Dioxolan der Formel (II) im allgemeinen 1,0 bis 5,0 Mol, vorzugsweise 1,0 bis 3 Mol an Amin der Formel (II) und gegebenenfalls 1,0 bis 5,0 Mol, vorzugsweise 1,0 bis 3,0 Mol an Reaktionshilfsmittel ein. Die Reaktionsdurchführung, Aufarbeitung und Isolierung der Reaktionsprodukte der Formel (I) erfolgt nach üblichen Methoden.

Als Verdünnungsmittel zur Durchführung des erfindungsgemäßen Verfahrens (b) kommen ebenfalls inerte organische Lösungsmittel infrage.

Hierzu gehören insbesondere aliphatische oder aromatische, gegebenenfalls halogenierte Kohlenwasserstoffe, wie beispielsweise Benzin, Petrolether, Hexan, Cyclohexan; Ether, wie Diethylether, Dioxan, Tetrahydrofuran oder Ethylenglykoldimethyl-oder diethylether; Ester, wie Essigsäureethylester oder Alkohole, wie Methanol, Ethanol oder Isopropanol.

Als Katalysator zur Durchführung des erfindungsgemäßen Verfahrens (b) können alle üblichen Hydrierkatalysatoren eingesetzt werden. Vorzugsweise verwendet man Edelmetallkatalysatoren, wie z.B. Platin oder Palladium, gegebenenfalls auf einem geeigneten Trägerstoff, wie z.B. Kohle.

Die Reaktionstemperaturen können bei der Durchführung des erfindungsgemäßen Verfahrens (b) in einem größeren Bereich variiert werden. Im allgemeinen arbeitet man bei Temperaturen zwischen 20 °C und 180 °C, vorzugsweise bei Temperaturen zwischen 60 °C und 150 °C.

Das erfindungsgemäße Verfahren (b) wird üblicherweise unter erhöhtem Druck durchgeführt. Im allgemeinen arbeitet man in Druckbereichen zwischen 1 und 250 atü, vorzugsweise zwischen 50 und 150 atü.

Zur Durchführung des erfindungsgemäßen Verfahrens (b) setzt man pro Mol an substituierten Aminoketal der Formel (Ia) im allgemeinen 1,0 bis 50 g, vorzugsweise 20 bis 30 g an Hydrierkatalysator zu. Die Reaktionsdurchführung, Aufarbeitung und Isolierung der Reaktionsprodukte erfolgt nach üblichen Methoden.

Zur Herstellung von pflanzenverträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt-und der I. und II. sowie IV. bis VIII. Nebengruppe infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten:
Halogenwasserstoffsäuren, wie z.B. Chlorwasserstoffsäure und Bromwasserstoffsäure, ferner Phosphorsäure, Salpetersäure und Schwefelsäure.
Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea

(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Braunspelzigkeit des Weizens (Leptosphaeria nodorum), gegen den Erreger der Braunfleckenkrankheit der Gerste (Cochliobolus sativus), gegen den Erreger der Netzfleckenkrankheit der Gerste (Pyrenophora teres) oder gegen Mehltauarten einsetzen. Daneben zeigen die erfindungsgemäßen Wirkstoffe auch sehr gute Wirkung gegen Pflanzenkrankheiten im Obst-und Gemüsebau, wie beispielsweise gegen den Erreger der Kartoffel-und Tomatenfäule (Phytophthora infestans), gegen den Erreger des Apfelschorfes (Venturia inaequalis) oder gegen Cercospora-Arten.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Sili kate; als feste Trägerstoffe für Granulate kommen in Frage: z.B. gebrochene und fraktionierte natürliche Gesteine wie Calcit, Marmor, Bims, Sepiolith, Dolomit, sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetate, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

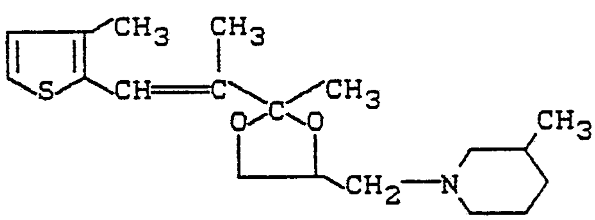

(Verfahren a)

Eine Mischung aus 19 g (0,07 Mol) 4-Chlormethyl-2-methyl-2-[1-(3-methylthien-2-yl)-propen-2-yl]-dioxolan und 18 g (0,18 Mol) 3-Methylpiperidin wird unter Rühren 16 Stunden auf 120 °C erhitzt. Zur Aufarbeitung wird die erkaltete Reaktionsmischung zwischen Wasser und Diethylether verteilt, die organische Phase abgetrennt, über Magnesiumsulfat getrocknet und im Vakuum eingedampft.

Man erhält 19 g (81 % der Theorie) an 2-Methyl-4-(3-methylpiperidin-1-yl-methyl)-2-[1-(3-methylthien-2-yl)-propen-2-yl]-dioxolan vom Brechungsindex $n_D^{20}$ 1.5402.

Herstellung der Ausgangsverbindung:

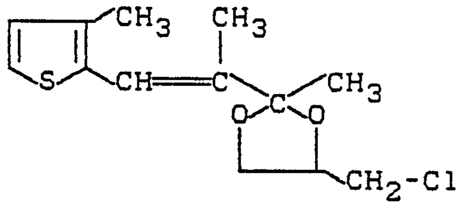

78 g (0,43 Mol) 2-Methyl-1-(3-methylthien-2-yl)-buten-3-on und 100 g (0,9 Mol) 3-Chlor-propan-1,2-diol in 600 ml Toluol werden unter Zusatz von 4 g saurem Ionenaustauscher (Lewasorb A10) 16 Stunden unter Rückfluß über einem Wasserabscheider erhitzt. Zur Aufarbeitung filtriert man die erkaltete Reaktionsmischung, wäscht das Filtrat mit Wasser, trocknet über Natriumsulfat und entfernt das Lösungsmittel im Vakuum.

Man erhält 77g (65 % der Theorie) an 4-Chlormethyl-2-methyl-2-[1-(3-methylthien-2-yl)-propen-2-yl]-dioxolan vom Brechungsindex $n_D^{20}$ 1.5430.

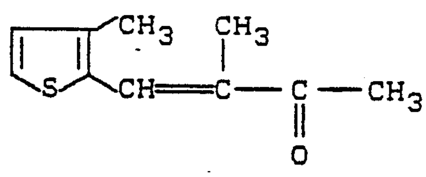

Zu einer Mischung aus 100 g (0,79 Mol) 3-Methylthiophen-2-aldehyd und 300 ml absolutem Methylethylketon gibt man 100 ml einer Lösung von Chlorwasserstoff in Ether (ca. 9 %ig) und rührt 24 Stunden bei Raumtemperatur. Zur Aufarbeitung entfernt man die flüchtigen Bestandteile im Vakuum und kristallisiert den Rückstand aus Ether um.

Man erhält 82 g (57 % der Theorie) an 2-Methyl-2-(3-methylthien-2-yl)-buten-3-on vom Schmelzpunkt 72 °C.

Beispiel 2:

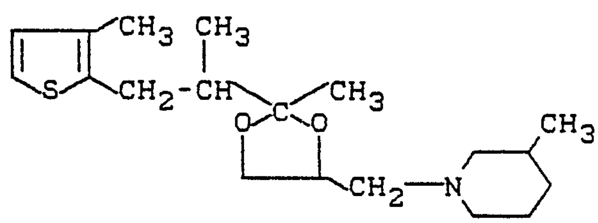

(Verfahren b)

15 g (0,05 Mol) 2-Methyl-4-(3-methylpiperidin-1-yl-methyl)-2-[1-(3-methylthien-2-yl)-propen-2-yl]-dioxolan in 150 ml Methanol werden mit 10 g Palladium auf Kohle (5 %ig) versetzt und 6 Stunden bei 80 °C und einem Druck von 120 bar hydriert. Zur Aufarbeitung filtriert man den Katalysator ab und entfernt das Lösungsmittel im Vakuum.

Man erhält 14,5 g (96 % der Theorie) an 2-Methyl-4-(3-methylpiperidin-1-yl-methyl)-2-[1-(3-methylthien-2-yl)-propan-2-yl]-dioxolan vom Brechungsindex $n_D^{20}$ 1.5139.

In entsprechender Weise und gemäß den allgemeinen Angaben zur Herstellung erhält man die folgenden substituierten Dioxolane der allgemeinen Formel (I):

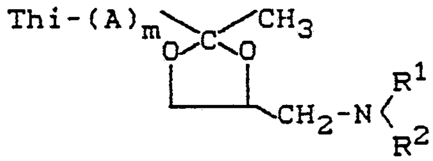 (I)

| Bsp. Nr. | Thi | A | m | 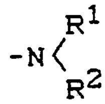 | Schmelzpunkt [°C] bzw. Brechungsindex [$n_D^{20}$] |
|---|---|---|---|---|---|
| 3 | 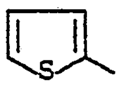 | 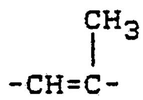 | 1 | 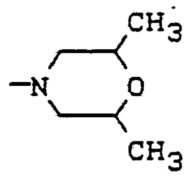 | 1.5381 |
| 4 | 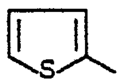 | 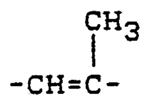 | 1 | 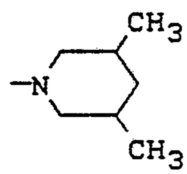 | 1.5328 |
| 5 | 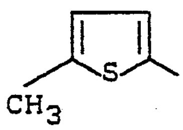 | 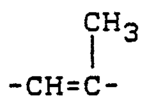 | 1 | 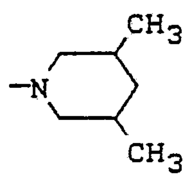 | 1.5397 |
| 6 | 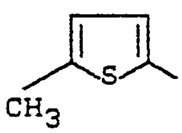 | 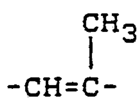 | 1 | 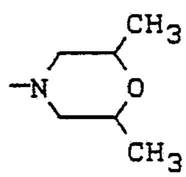 | 1.5402 |
| 7 | 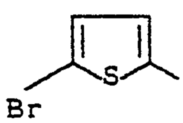 | 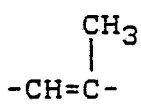 | 1 | 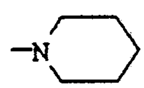 | 1.5711 |
| 8 | 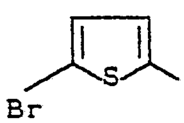 | 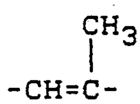 | 1 | 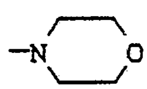 | 1.5697 |

| Bsp. Nr. | Thi | A | m | 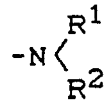 | Schmelzpunkt [°C] bzw. Brechungsindex [$n_D^{20}$] |
|---|---|---|---|---|---|
| 9 | 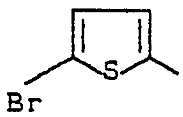 | 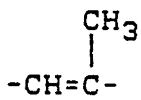 | 1 | 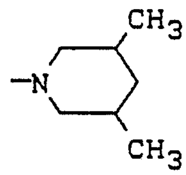 | 1,5552 |
| 10 | 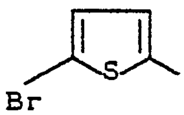 | 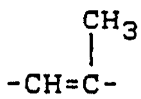 | 1 | 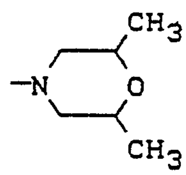 | 1,5661 |
| 11 | 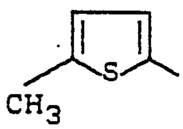 | 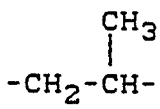 | 1 | 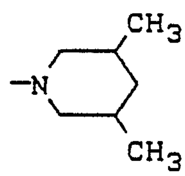 | 1,5063 |
| 12 | 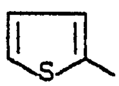 | - | 0 | 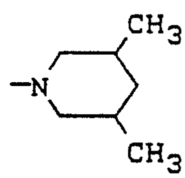 | 1,5096 |
| 13 | 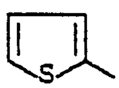 | - | 0 | 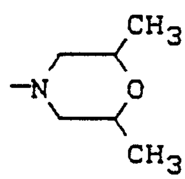 | 1,5672 |
| 14 | 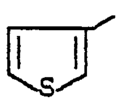 | - | 0 | 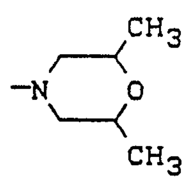 | 1,5322 |
| 15 | 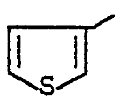 | - | 0 | 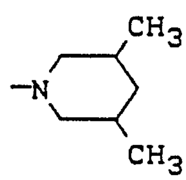 | 1,5141 |

| Bsp. Nr. | Thi | A | m | 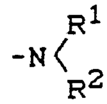 | Schmelzpunkt [°C] bzw. Brechungsindex [$n_D^{20}$] |
|---|---|---|---|---|---|
| 16 | 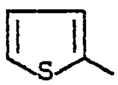 | 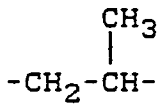 | 1 | 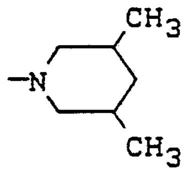 | Fp 35° C |
| 17 | 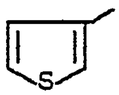 | 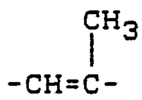 | 1 | 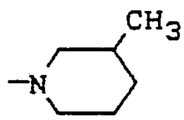 | 1,5383 |
| 18 | 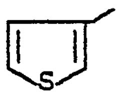 | 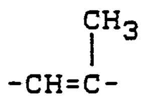 | 1 | 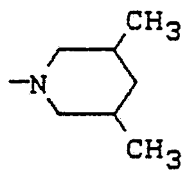 | 1,5334 |
| 19 | 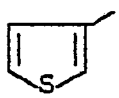 | 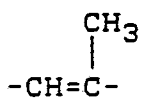 | 1 | 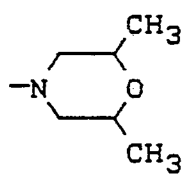 | 1,5408 |
| 20 | 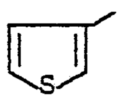 | 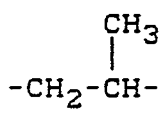 | 1 | 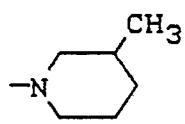 | 1,5146 |
| 21 | 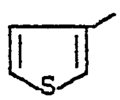 | 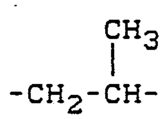 | 1 | 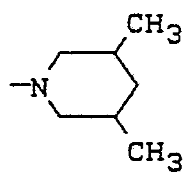 | 1,5132 |
| 22 | 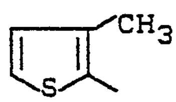 | 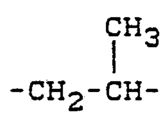 | 1 | 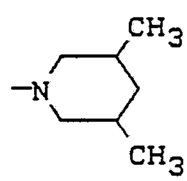 | 1,5256 |

| Bsp. Nr. | Thi | A | m | 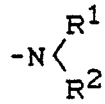 | Schmelzpunkt [°C] bzw. Brechungsindex [$n_D^{20}$] |
|---|---|---|---|---|---|
| 23 | 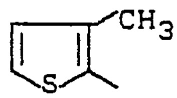 | 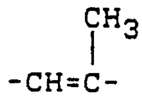 | 1 | 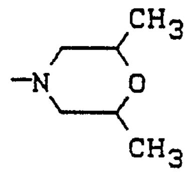 | 1.5376 |
| 24 | 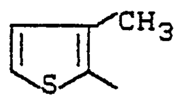 | 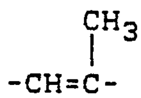 | 1 | 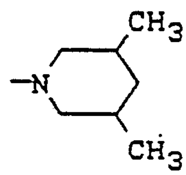 | 1.5354 |
| 25 | 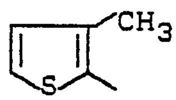 | 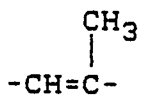 | 1 | 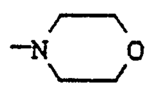 | 1.5346 |
| 26 | 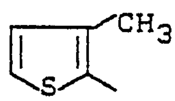 | 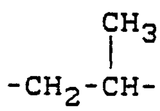 | 1 | 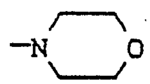 | 1.5091 |

Anwendungsbeispiele

In den folgenden Anwendungsbeispielen wurden die nachstehend aufgeführten Verbindungen als Vergleichssubstanzen eingesetzt:

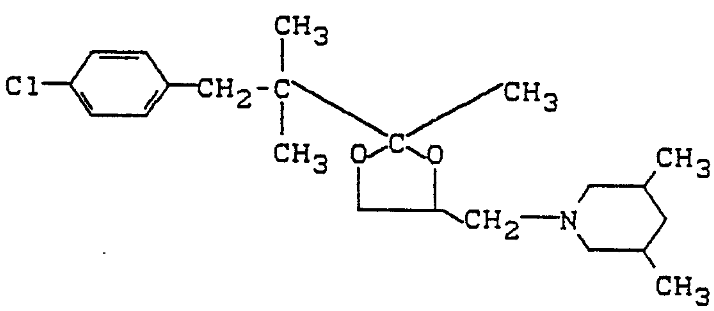

(A)

2-[1-(4-Chlorphenyl)-2-methylprop-2-yl]-4-(3,5-dimethylpiperidin-1-yl-methyl)-2-methyl-dioxolan und

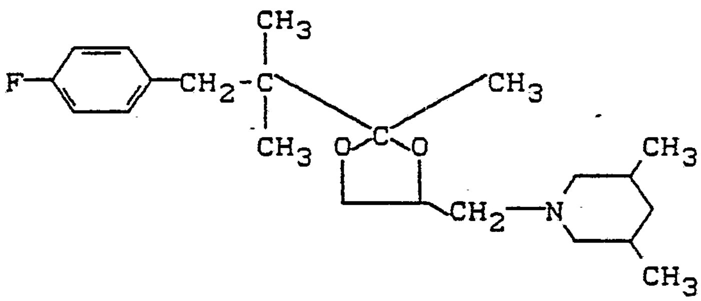

(B)

2-[1-(4-Fluorphenyl)-2-methylprop-2-yl]-4-(3,5-dimethylpiperidin-1-yl-methyl)-2-methyl-dioxolan (beide bekannt aus EP 97 822)

Beispiel A

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßig Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 9, 10, 17, 18, 19, 20 und 21.

## Ansprüche

1. Substituierte Aminoketale der allgemeinen Formel (I),

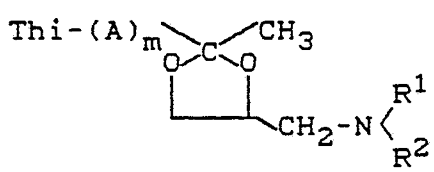

(I)

in welcher
Thi für gegebenenfalls substituiertes Thienyl steht,
A für eine Alkylen-oder Alkenylenkette steht,
m für eine Zahl 0 oder 1 steht,
$R^1$ für Wasserstoff, Alkyl oder Alkenyl steht und
$R^2$ für Alkyl oder Alkenyl steht oder
$R^1$ und $R^2$ gemeinsam mit dem Stickstoffatom, an welches sie gebunden sind, für einen Rest

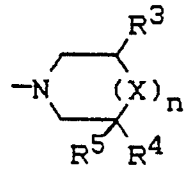

stehen, wobei
$R^3$, $R^4$ und $R^5$ unabhängig voneinander jeweils für Wasserstoff, Alkyl oder Hydroxyalkyl stehen,
X für Sauerstoff, Schwefel, eine Methylen-oder eine Ethylengruppe steht und
n für eine Zahl 0 oder 1 steht,
sowie deren pflanzenverträgliche Säureadditionssalze und Metallsalzkomplexe.

2. Substituierte Aminoketale gemäß Anspruch 1, in denen in der Formel (I)
Thi für gegebenenfalls ein-oder zweifach, gleich oder verschieden substituiertes Thienyl steht, wobei als Substituenten genannt seien: Halogen, Nitro, jeweils geradkettiges oder verzweigtes Alkyl, Halogenalkyl oder Halogenalkoxy mit jeweils 1 bis 4 Kohlenstoffatomen und gegebenenfalls 1 bis 9 gleichen oder verschiedenen Halogenatomen,
A für eine jeweils geradkettige oder verzweigte Alkylenkette oder Alkenylenkette mit jeweils 3 bis 6 Kohlenstoffatomen steht,
m für eine Zahl 0 oder 1 steht,
$R^1$ für Wasserstoff oder für jeweils geradkettiges oder verzweigtes Alkyl oder Alkenyl mit jeweils bis zu 12 Kohlenstoffatomen steht und
$R^2$ jeweils geradkettiges oder verzweigtes Alkyl oder Alkenyl mit jeweils bis zu 12 Kohlenstoffatomen steht oder
$R^1$ und $R^2$ gemeinsam mit dem Stickstoffatom, an welches sie gebunden sind, für einen Rest

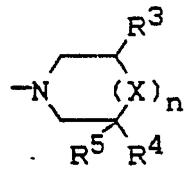

stehen, wobei$R^3$, $R^4$ und $R^5$ unabhängig voneinander jeweils für Wasserstoff oder für jeweils geradkettiges oder verzweigtes Alkyl oder Hydroxyalkyl mit jeweils 1 bis 4 Kohlenstoffatomen stehen,
X für Sauerstoff, Schwefel, eine Methylenoder eine Ethylengruppe steht und
n für eine Zahl 0 oder 1 steht.

3. Substituierte Aminoketale gemäß Anspruch 1, in denen in der Formel (I)
Thi für gegebenenfalls ein-oder zweifach, gleich oder verschieden substituiertes 2-oder 3-Thienyl steht, wobei als Substituenten genannt seien: Fluor, Chlor, Brom, Nitro, Methyl, Ethyl, n-oder i-Propyl, n-, i-, s-oder t-Butyl,

A für eine der Gruppen 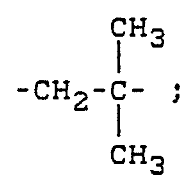 ; 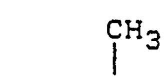 ;

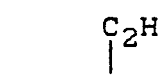 ; 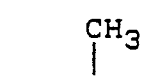 oder 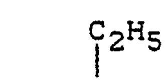 steht,

m für Zahl 0 oder 1 steht,
$R^1$ für Wasserstoff oder für jeweils geradkettiges oder verzweigtes Alkyl oder Alkenyl mit jeweils bis zu 6 Kohlenstoffatomen steht und
$R^2$ für jeweils geradkettiges oder verzweigtes Alkyl oder Alkenyl mit jeweils bis zu 6 Kohlenstoffatomen

steht.

4. Substituierte Aminoketale gemäß Anspruch 1, in denen in der Formel (I)
Thi für gegebenenfalls ein-oder zweifach, gleich oder verschieden substituiertes 2-oder 3-Thienyl steht, wobei als Substituienten genannt seien: Fluor, Chlor, Brom, Nitro, Methyl, Ethyl, n-oder i-Propyl, n-, i-, s-oder t-Butyl,

A für eine der Gruppen 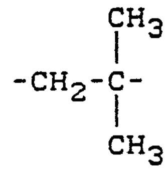 ; 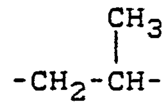;

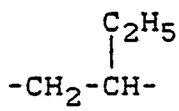 ; 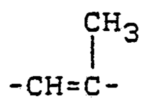 oder 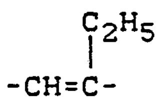 steht,

m für eine Zahl 0 oder 1 steht und
$R^1$ und $R^2$ gemeinsam mit dem Stickstoffatom, an welches sie gebunden sind, für einen Rest

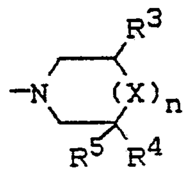

stehen, wobei
$R^3$, $R^4$ und $R^5$ unabhängig voneinander jeweils für Wasserstoff, Methyl, Ethyl oder Hydroxymethyl stehen,
X für Sauerstoff, Schwefel, eine Methylengruppe oder eine Ethylengruppe steht und
n für eine Zahl 0 oder 1 steht.

5. Verfahren zur Herstellung von substituierten Aminoketalen der Formel (I),

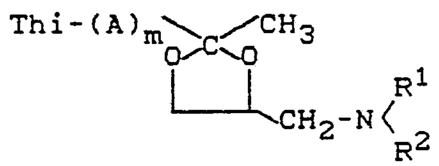 (I)

in welcher
Thi für gegebenenfalls substituiertes Thienyl steht,
A für eine Alkylen-oder Alkenylenkette steht,
m für eine Zahl 0 oder 1 steht,
$R^1$ für Wasserstoff, Alkyl oder Alkenyl steht und
$R^2$ für Alkyl oder Alkenyl steht oder
$R^1$ und $R^2$ gemeinsam mit dem Stickstoffatom, an welches sie gebunden sind, für einen Rest

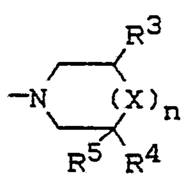

stehen, wobei
$R^3$, $R^4$ und $R^5$ unabhängig voneinander jeweils für Wasserstoff, Alkyl oder Hydroxyalkyl stehen,

X für Sauerstoff, Schwefel, eine Methylen-oder eine Ethylengruppe steht und
n für eine Zahl 0 oder 1 steht,
sowie deren pflanzenverträgliche Säureadditionssalze und Metallsalzkomplexe, dadurch gekennzeichnet, daß man
(a) Dioxolane der Formel (II)

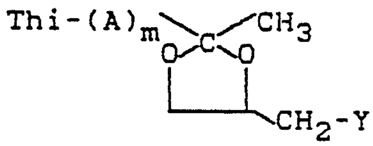

(II)

in welcher
Y für eine elektronenanziehende Abgangsgruppe steht und
Thi, A und m die oben angegebene Bedeutung haben,
mit Aminen der Formel (III),

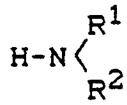

(III)

in welcher
$R^1$ und $R^2$ die oben angegebene Bedeutung haben,
gegebenenfalls in Gegenwart eines Verdünnungsmittels und gegebenenfalls in Gegenwart eines Reaktionshilfsmittels umsetzt oder daß man
(b) die mit Hilfe des Verfahrens (a) erhältlichen substituierten Aminoketale der Formel (Ia),

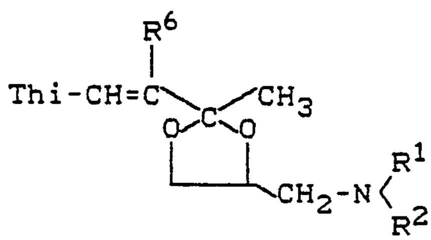

(Ia)

in welcher
$R^6$ für Wasserstoff oder Alkyl steht und
Thi, $R^1$ und $R^2$ die oben angegebene Bedeutung haben,
mit Wasserstoff in Gegenwart eines Katalysators und in Gegenwart eines Verdünnungsmittels hydriert und gegebenenfalls anschließend eine Säure oder ein Metallsalz addiert.

6. Schädlingsbekämpfungsmittel, gekennzeichnet durch einen Gehalt an mindestens einer Verbindung der allgemeinen Formel (I) gemäß den Ansprüchen 1 oder 5.

7. Verfahren zur Bekämpfung von Schädlingen, dadurch gekennzeichnet, daß man mindestens eine Verbindung der Formel (I) gemäß den Ansprüchen 1 oder 5 auf Schädlinge und/oder ihren Lebensraum einwirken läßt.

8. Verwendung von Verbindungen der allgemeinen Formel (I) gemäß den Ansprüchen 1 oder 5 zur Bekämpfung von Schädlingen.

9. Verwendung von Verbindungen der allgemeinen Formel (I) gemäß Anspruch 8 zur Bekämpfung von pilzlichen Schädlingen.

10. Verfahren zur Herstellung von Schädlingsbekämpfungsmitteln, dadurch gekennzeichnet, daß man Verbindungen der allgemeinen Formel (I) gemäß den Ansprüchen 1 oder 5 mit Streckmitteln und/oder oberflächenaktiven Mitteln vermischt.

11. Dioxolane der Formel (II)

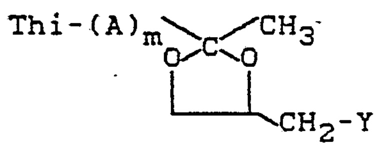

(II)

in welcher
Thi für gegebenenfalls substituiertes Thienyl steht,
A für eine Alkylen-oder Alkenylenkette steht,
m für Zahl 0 oder 1 steht und
Y für eine elektronenanziehende Abgangsgruppe steht.

12. Verfahren zur Herstellung von Dioxolanen der Formel (II),

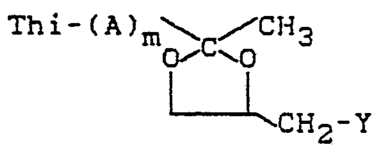

(II)

in welcher
Thi für gegebenenfalls substituiertes Thienyl steht,
A für eine Alkylen-oder Alkenylenkette steht,
m für eine Zahl 0 oder 1 steht und
Y für eine elektronenanziehende Abgangsgruppe steht, dadurch gekennzeichnet, daß man
a) Ketone der Formel (IV),

$Thi-(A)_m-\underset{\overset{\|}{O}}{C}-CH_3$ (IV)

in welcher
Thi, A und m die oben angegebene Bedeutung haben mit Diolen der Formel (V),

$HO-CH_2-\overset{\overset{OH}{|}}{C}H-CH_2-Y^1$ (V)

in welcher,
$Y^1$ für Hydroxy oder Halogen steht,
gegebenenfalls in Gegenwart eines Verdünnungsmittels und gegebenenfalls in Gegenwart eines Katalysators, bei Temperaturen zwischen 50°C und 120°C umsetzt, und
b) daß man in den Fällen, wo $Y^1$ in der Formel (V) für eine Hydroxygruppe steht, in einer 2. Stufe die so erhältlichen Hydroxymethyldioxolane der Formel (VI),

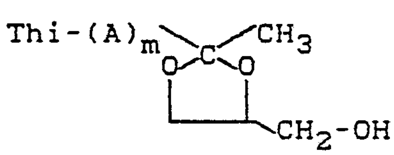

(VI)

in welcher
Thi, A und m die oben angegebene Bedeutung haben,
mit gegebenenfalls substituierten Alkyl-oder Arylsulfonylhalogeniden der Formel (VII),
$Z-SO_2-Hal$ (VII)
in welcher
Hal für Halogen steht und
Z für jeweils gegebenenfalls substituiertes Alkyl oder Aryl steht,
gegebenenfalls in Gegenwart eines Verdünnungsmittels und gegebenenfalls in Gegenwart eines Säurebindemittels bei Temperaturen zwischen -20°C und +100°C umsetzt.

Europäisches Patentamt

# EUROPÄISCHER RECHERCHENBERICHT

Nummer der Anmeldung

EP 87 11 4816

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 133 950 (BAYER)<br>* Ansprüche *<br>--- | 1,5-10 | C 07 D 409/14<br>C 07 D 409/06<br>C 07 D 413/14<br>A 01 N 43/28<br>A 01 N 43/40<br>A 01 N 43/84 |
| A | EP-A-0 167 768 (BAYER)<br>* Ansprüche *<br>--- | 1,5-10 | |
| D,A | EP-A-0 097 822 (BAYER)<br>* Ansprüche *<br>----- | 1,5-10 | |

RECHERCHIERTE SACHGEBIETE (Int. Cl.4)

A 01 N 43/00
C 07 D 409/00
C 07 D 413/00
C 07 D 405/00
C 07 D 317/00

Der vorliegende Recherchenbericht wurde für alle Patentansprüche erstellt

| Recherchenort | Abschlußdatum der Recherche | Prüfer |
|---|---|---|
| DEN HAAG | 22-01-1988 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)